# EUROPEAN PATENT SPECIFICATION

(11) **EP 4 150 632 B1**
(45) Date of publication and mention of the grant of the patent: **29.04.2026**
(21) Application number: 21724621.4
(22) Date of filing: 06.05.2021
(51) Int. Cl.: G16H 50/20, G16H 30/40, A61B 5/341, A61B 5/346, A61B 5/00, G16H 50/70

(54) **TIME SERIES DATA CONVERSION FOR MACHINE LEARNING MODEL APPLICATION**
ZEITREIHENDATENUMWANDLUNG FÜR MASCHINENLERNMODELLANWENDUNG
CONVERSION DE DONNÉES CHRONOLOGIQUES POUR APPLICATION DE MODÈLE D'APPRENTISSAGE AUTOMATIQUE

(30) Priority: 11.05.2020 US 202063022752 P
(43) Date of publication of application: 22.03.2023
(73) Proprietor: Koninklijke Philips N.V., 5656 AG Eindhoven (NL)
(72) Inventor: BABAEIZADEH, Saeed, 5656 AE Eindhoven (NL)
(74) Representative: Philips Intellectual Property & Standards
(86) International application number: PCT/EP2021/061962
(87) International publication number: WO 2021/228672

(56) References cited:
- US-A1- 2018 350 468
- AL RAHHAL MOHAMAD MAHMOUD ET AL: "Dense Convolutional Networks With Focal Loss and Image Generation for Electrocardiogram Classification", IEEE ACCESS, vol. 7, 26 December 2019 (2019-12-26), pages 182225 - 182237, XP011762938, DOI: 10.1109/ACCESS.2019.2960116
- IZCI ELIF ET AL: "Cardiac Arrhythmia Detection from 2D ECG Images by Using Deep Learning Technique", 2019 MEDICAL TECHNOLOGIES CONGRESS (TIPTEKNO), IEEE, 3 October 2019 (2019-10-03), pages 1 - 4, XP033649810, DOI: 10.1109/TIPTEKNO.2019.8895011
- BRAUN TILL ET AL: "Detection of myocardial ischemia due to clinically asymptomatic coronary artery stenosis at rest using supervised artificial intelligence-enabled vectorcardiography - A five-fold cross validation of accuracy", JOURNAL OF ELECTROCARDIOLOGY, ELSEVIER SCIENCE, XX, vol. 59, 8 January 2020 (2020-01-08), pages 100 - 105, XP086116012, ISSN: 0022-0736, [retrieved on 20200108], DOI: 10.1016/J.JELECTROCARD.2019.12.018
- DEHNAVI ALI REZA MEHRI ET AL: "Detection and classification of cardiac ischemia using vectorcardiogram signal via neural network", JOURNAL OF RESEARCH IN MEDICAL SCIENCES, 28 February 2011 (2011-02-28), pages 136 - 142, XP055829976, Retrieved from the Internet <URL:https://www.ncbi.nlm.nih.gov/pmc/articles/PMC3214294/?report=printable> [retrieved on 20210803]

## Description

### Technical Field

Various embodiments described herein are directed generally to health care and/or artificial intelligence. More particularly, but not exclusively, various methods and apparatus disclosed herein relate to converting time series data, such as electrocardiogram ("ECG") waveforms, into different forms suitable for application across machine learning models.

### Background

Modern artificial intelligence techniques such as deep learning have numerous applications. Image processing may be one of the most developed. Numerous machine learning models (e.g., convolutional neural networks) already exist for performing image processing on digital images to perform tasks such as segmentation, object recognition, handwriting recognition, facial recognition, *etc.* In their most basic and/or common forms, many of these models do not operate on time-series data, i.e., a sequence of inputs that each corresponds to a point in time. Consequently, they may be adaptable for use across a variety of domains.

Techniques exist for processing time-series data such as electrocardiogram ("ECG") waveforms to perform tasks like clinical decision making, diagnosis, interpretation, reduction of medical errors, expedition of patient care, *etc.* However, artificial intelligence models that operate on time-series data, such as recurrent neural networks, are more complex than models that operate on one-off data such as feed-forward neural networks. This added complexity makes them more difficult to train, as well as less adaptable across domains.

By way of further background, reference is made to the papers recited below:
AL RAHHAL MOHAMAD MAHMOUD ET AL: "Dense Convolutional Networks With Focal Loss and Image Generation for Electrocardiogram Classification", IEEE ACCESS, vol. 7, 26 December 2019 (2019-12-26), pages 182225-182237. This paper discloses to apply dense convolutional networks for the classification of electrocardiogram signals by converting the electrocardiogram signals into an image.
IZCI ELIF ET AL: "Cardiac Arrhythmia Detection from 20 ECG Images by Using Deep Learning Technique", 2019 MEDICAL TECHNOLOGIES CONGRESS (TIPTEKNO), IEEE, 3 October 2019 (2019-10-03), pages 1-4.
BRAUN TILL ET AL: "Detection of myocardial ischemia due to clinically asymptomatic coronary artery stenosis at rest using supervised artificial intelligence-enabled vectorcardiography - A five-fold cross validation of accuracy", JOURNAL OF ELECTROCARDIOLOGY, ELSEVIER SCIENCE, XX, vol. 59, 8 January 2020, pages 100-105.
Dehnavi Ali Reza Mehri ET AL: "Detection and classification of cardiac ischemia using vectorcardiogram signal via neural network", Journal of Research in Medical Sciences, 28 February 2011, pages 136-142.

### Summary

The present disclosure is directed to methods and apparatus for converting time series data such as electrocardiogram ("ECG") data into forms suitable for application across machine learning models. For example, in various embodiments, ECG data may be obtained from a subject using various numbers of leads, such as twelve leads, or another number of leads. This ECG data may then be converted using techniques described herein to a form that is suitable for application across a machine learning model such as a convolutional neural network ("CNN"). This form may preserve both temporal and spatial information contained in the ECG data. Put another way, the converted form may preserve both the rhythm and morphology of ECG waveforms.

For example, ECG data (e.g., waveform(s)) may be used to generate a two dimensional digital image that preserves both the rhythm and morphology of the ECG data. In some cases this two-dimensional image may include multiple layers or channels corresponding to, for instance, different colors. For example, one type of digital image may include, for each pixel, a red channel, a green channel, and a blue channel ("RGB"). Other combinations of channels are contemplated. As will be described in more detail herein, various spatial and/or temporal aspects of the ECG data may be encoded into these different channels of the two-dimensional data. The two-dimensional digital image may then be applied as input across a suitably-trained machine learning model *(e.g.,* a CNN) to generate output indicative of a health condition of the subject from which the ECG data was obtained. Additionally, before the machine learning model is trained, similar two-dimensional data that is labeled by medical personnel may be used to train the machine learning model.

In some implementations, transfer learning may be employed to expedite the process of training the machine learning model by leveraging at least part of a preexisting, pre-trained machine learning model. Various pre-trained machine learning models are widely available for tasks such as image and/or object classification. For example, some preexisting models are trained to identify and/or classify, for instance, objects like different types of animals, furniture, tools, vehicles, *etc.* Others may be trained to perform tasks such as handwriting recognition. In some embodiments, these preexisting models-and particularly their "upstream" hidden layers that identify/classify relatively abstract visual features-may be leveraged to train additional downstream hidden layers that identify aspects of two-dimensional digital images generated from time-series data using techniques described herein.

Various techniques may be employed to convert time series data such as ECG data to two-dimensional image data. For example, in some embodiments, the ECG data may take the form of twelve waveforms that correspond to each of twelve-lead ECG data. Vectorcardiography ("VCG") data may be generated from these ECG data, or from an intermediate form of these data. In addition to calculating VCG leads from the standard ECG lead system, VCG leads may also be recorded directly. Since in standard twelve-lead ECGs, four waveforms may be themselves derived from the other eight waveforms, in some embodiments, VCG leads are derived using only the eight waveforms. It may also be possible to derive VCGs from other ECG lead systems that may have fewer or more than eight leads. In some embodiments, each of the twelve waveforms may be converted first into a respective single representative beat, *e.g.,* an average or mean. The twelve representative beats may then be converted into three VCG beats, with each VCG beat corresponding to a heart vector in one dimension of three-dimensional ("3D") space. Three VCG projections may then be determined, with each VCG projection representing a respective one of the three VCG beats on a spatial plane. These projections may be combined into a two-dimensional image, *e.g.,* with each VCG projection being stored in a respective channel *(e.g.,* RGB) of the two-dimensional image.

The invention is defined by the appended independent claims.

### Brief Description of the Drawings

In the drawings, like reference characters generally refer to the same parts throughout the different views. Also, the drawings are not necessarily to scale, emphasis instead generally being placed upon illustrating various principles of the embodiments described herein.
Fig. 1 illustrates an example environment in which selected aspects of the present disclosure may be implemented.
Fig. 2 depicts example twelve-lead ECG data and representative beats generated therefrom.
Fig. 3 depicts one example of how the representative beats from Fig. 2 can be converted into some number (e.g., three) of VCG beats.
Fig. 4 depicts one example of how the VCG beats from Fig. 3 may be used to generate a multi-channel two-dimensional image.
Fig. 5 demonstrates one example of how transfer learning may be employed to train aspects of an existing machine learning model to detect health conditions.
Fig. 6 depicts an example method for practicing selected aspects of the present disclosure.
Fig. 7 depicts an example computer architecture.
Fig. 8 depicts a confusion matrix that reveals accuracy achieved using techniques described herein.

### Detailed Description

Modern artificial intelligence ("AI") techniques such as deep learning have numerous applications, and image processing may be one of the most developed. While relatively adaptable across domains, these deep learning models may not be configured to processing time-series data such as electrocardiogram ("ECG") waveforms. Moreover, AI models that process time-series data are more complex, less readily available, and even when available, are not easily adapted for new domains. In view of the foregoing, various embodiments and implementations of the present disclosure are directed to converting time series data, such as ECG waveforms, into forms suitable for application across non-time-series machine learning models.

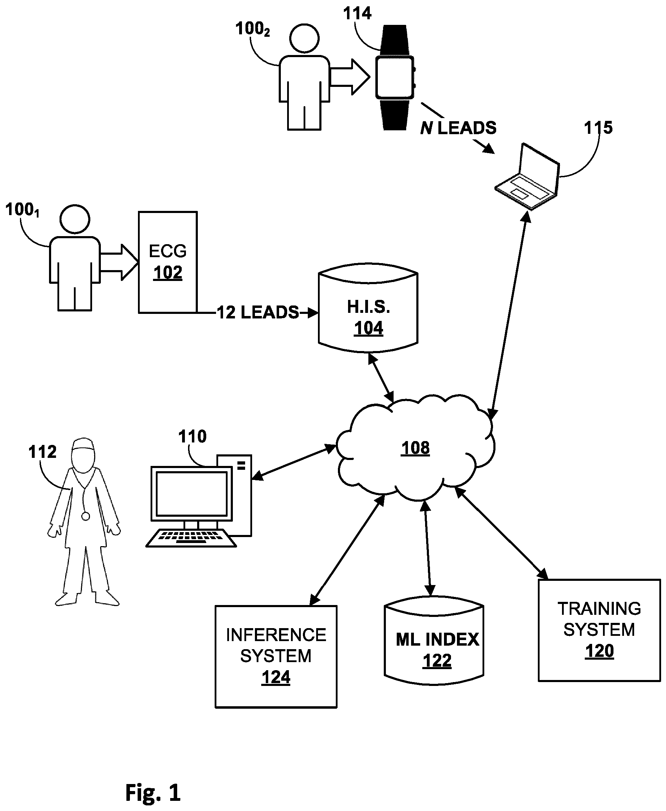

Fig. 1 depicts an example environment in which selected aspects of the present disclosure may be implemented, in accordance with various embodiments. The computing devices depicted in Fig. 1 may include, for example, one or more of: a desktop computing device, a laptop computing device, a tablet computing device, a mobile phone computing device, a computing device of a vehicle of the user (e.g., an in-vehicle communications system, an in-vehicle entertainment system, an in-vehicle navigation system), a standalone interactive speaker (which in some cases may include a vision sensor), a smart appliance such as a smart television (or a standard television equipped with a networked dongle with automated assistant capabilities), and/or a wearable apparatus of the user that includes a computing device *(e.g.,* a watch of the user having a computing device, glasses of the user having a computing device, a virtual or augmented reality computing device). Additional and/or alternative computing devices may be provided.

In Fig. 1, a first subject 100₁ is being monitored by an ECG device 102, *e.g.,* at a hospital, to obtain time series data in the form of ECG data. For example, this ECG data may include twelve-lead ECG data as illustrated in Fig. 1. This ECG data may be provided to and/or stored in a hospital information system ("HIS") 104 or another similar healthcare system, e.g., as part of an electronic health record ("EHR") for the first subject 100₁. While the ECG data is provided directly to HIS 104 in Fig. 1, this is not meant to be limiting. In various embodiments, the ECG data may be provided to HIS 104 over one or more networks 108, which can include one or more local area networks and/or one or more wide area networks such as the Internet.

Techniques described herein are not limited to twelve-lead ECG data (or to ECG data at all, for that matter). For example, a second subject 100₂ is being monitored by a wearable ECG device in the form of a smart watch 114. This smart watch 114 may provide ECG data of a lesser number of leads, *e.g.,* one lead, to an intermediate computing device, such as a laptop 115 operated by second user 100₂. Laptop 115 in turn may provide this ECG data to HIS 104 via network(s) 108 (or in some cases smart watch 114 may provide the ECG data directly to HIS 104 via network(s) 108).

A training system 120 and an inference system 124 may be implemented using any combination of hardware and software in order to create, manage, and/or apply machine learning model(s) stored in a machine learning ("ML") model database 122. Training system 120 may be configured to apply training data such as two-dimensional images generated using techniques herein as input across one or more of the models in database 122 to generate output. The output generated using the training data may be compared to labels associated with the training data in order to determine error(s) associated with the model(s). A training example's label may indicate, for instance, the presence and/or probability of a health condition in a subject from which the training example was generated. These error(s) may then be used, e.g., by training system 120, to train the model(s) using techniques such as back propagation and gradient descent (stochastic or otherwise).

Inference system 124 may be configured to use the trained machine learning model(s) in database 122 to infer health conditions of subjects based on two-dimensional imagery generated using techniques described herein. In some embodiments, training system 120 and/or inference system 124 may be implemented as part of a distributed computing system that is sometimes referred to as the "cloud," although this is not required.

Fig. 1 also depicts health care personnel such as a doctor 112 that operates a computing device 110 in order to make inferences about health conditions of subjects (e.g., 100₁₋₂) as described herein. In particular, computing device 110 may be connected to network(s) 108 and thereby may interact with inference system 124 in order to make health condition inferences as described herein. For example, the doctor 112 may be able to make health condition inferences about first subject 100₁ based on the twelve-lead ECG data obtained from first subject 100₁. Similarly, doctor 112 may be able to make similar inferences about second subject 100₂ based on the single-lead ECG data obtained by smart watch 114.

In some embodiments, the ability to make these inferences may be provided as part of a software application that aids doctor 112 with diagnosis, e.g., a clinical decision support ("CDS") application. In some such embodiments, doctor 112 may rely on the inference as a "second opinion" to buttress or challenge their own medical opinion. Alternatively, the inferences may be used as ECG screening tests to separate, for instance, normal from abnormal ECG signals in a presumed healthy population *(e.g.,* students, athletes, soldiers, *etc.*) so that further investigation can be performed on those subjects having abnormal ECG signals. Additionally or alternatively, techniques described herein may be incorporated into medical equipment that also incorporates ECG signals, such as exercise stress testing machines, defibrillators, cardiographs, bedside monitors, and so forth.

In some embodiments, subjects 100₁₋₂ themselves may take advantage of disclosed techniques to determine likelihoods that they have health conditions, e.g., whether their heart beats are normal or abnormal. For example, second subject 100₂ may operate laptop 115 in order to interface with, and receive health condition inferences from, inference system 124. In some embodiments, e.g., to preserve privacy and/or respect privacy laws and regulations, one or more trained machine learning model(s) may be distributed from database 122 to remote computing devices (e.g., laptop 115). That way, the remote computing devices can perform selected aspects of the present disclosure on locally-obtained time-series data *(e.g.,* ECG data) without having to transport the ECG data to, for instance, the "cloud."

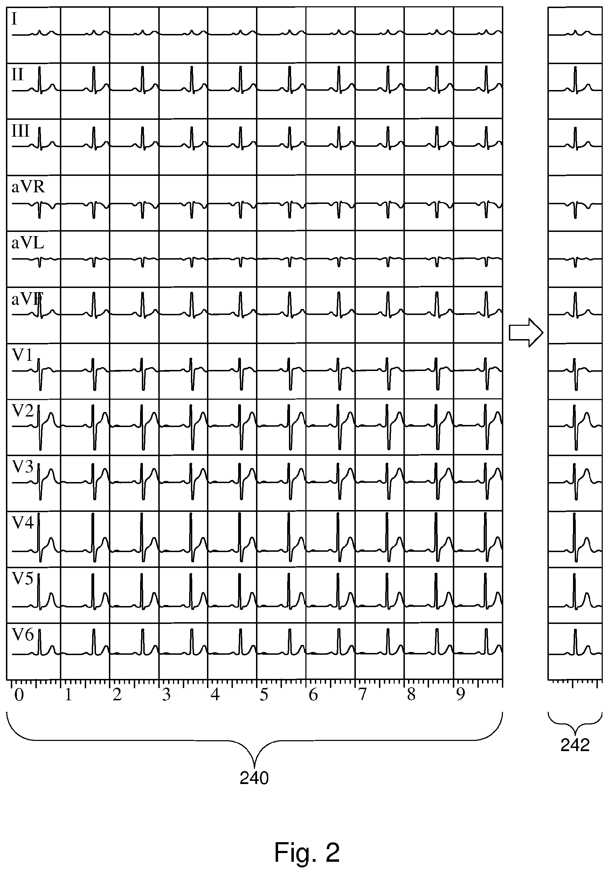

Figs. 2-4 depict one example of how ECG data (e.g., generated by ECG device 102 in Fig. 1) may be converted into a form-namely, a two-dimensional digital image having three channels *(e.g.,* red, green, and blue)-that is suitable for use with an image processing machine learning model such as a CNN. In this example, the ECG data takes the form of twelve-lead ECG data, but this is not meant to be limiting. ECG having less leads, such as single-lead ECG data generated by smart watch 114, may also be converted into VCG, and then into two dimensional imagery.

In Fig. 2, twelve-lead ECG data 240 is depicted at left, and includes I, II, III, aVR, aVL, aVF, and V1-V6. In this example, ten seconds of ECG waveforms are acquired, but other time intervals are possible. On the right in Fig. 2, at 242, each lead waveform has been converted to a single "representative" beat per lead. A representative beat may be, for instance, an average of all beats of that lead's waveform. This process attempts to preserve all of the detail of the ECG signal while mitigating random fluctuations generated by, for instance, muscle noise.

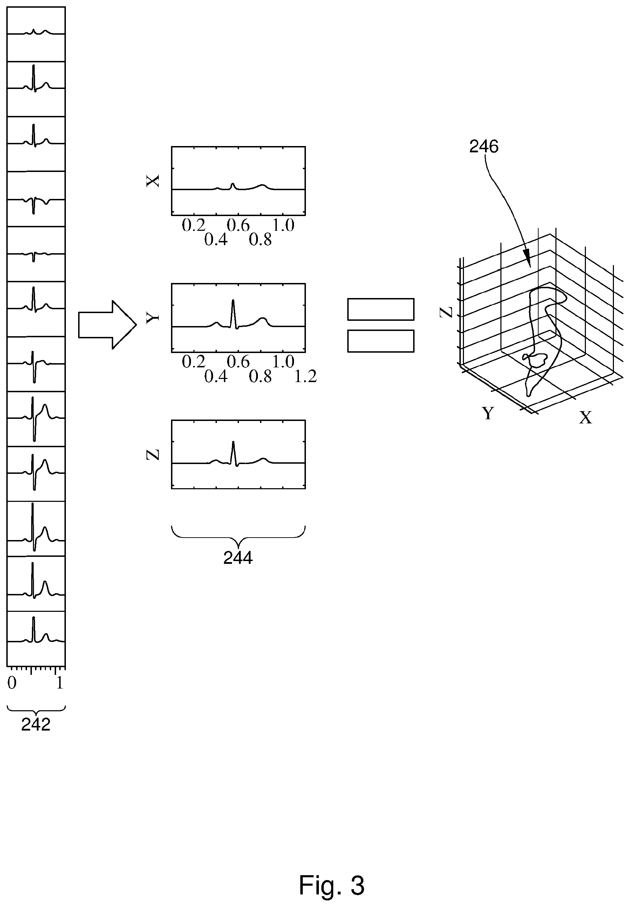

In Fig. 3, the twelve representative beats 242 are converted into some number of VCG beats 244. In Fig. 3, three VCG beats 244 are generated, one per dimension of a 3D space. In some embodiments, a three-dimensional VCG may be synthesized from the twelve-lead ECG 242 using matrix multiplication. Intuitively, to convert to VCG beats 244 involves recording a direction and magnitude of the electrical forces of the heart by means of a continuous series of vectors that form a curving line around a center in three-dimensional space, as shown at right 246 in Fig. 3. Thus, each VCG beat may represent the heart vector in one of three dimensions. In some embodiments, VCG leads may be recorded directly. In some embodiments, to improve spatial resolution, the VCG beats may be upsampled to, for instance, 1,000 samples per second, although this is not required.

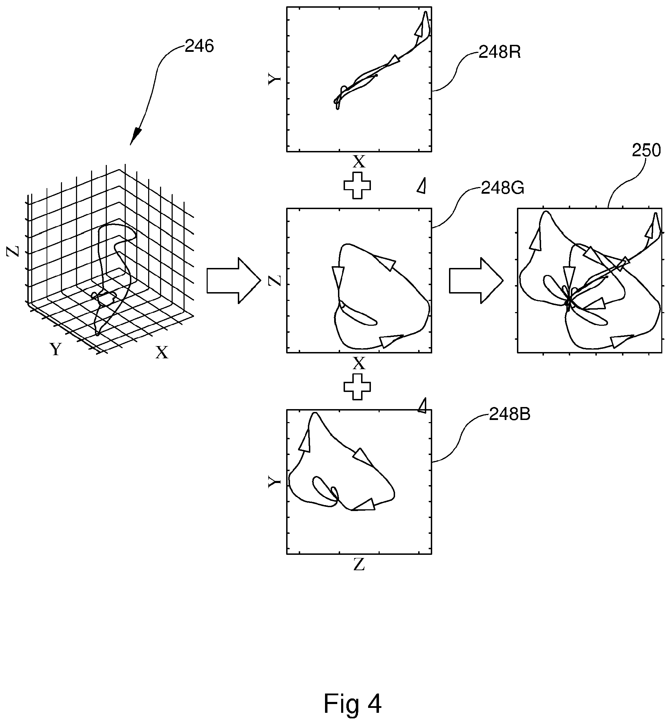

Fig. 4 shows how the VCG vector in three-dimensional space 246 may be converted into a two-dimensional digital image 250 with three channels. As one example, an RGB image may be stored as an *m*-by-*n*-by-3 data array that defines red, green, and blue color components for each individual pixel. The color of each pixel may be determined by the combination of the red, green, and blue intensities stored in each color plane at the pixel's location. In some embodiments, the VCG signal in three dimensions may be projected across three spatial planes, such that each projection can be used as one layer of an RGB image. For example, the projection 248R on the XY plane may be used as the red channel, the projection 248G on the XZ plane may be used as the green channel, and the projection 248B on the ZY plane may be used as the blue channel. When encoding these projections into RGB layers, in some embodiments, the projections may be rotated to reduce their overlap in the digital image 250. For consistency among different recordings, the degree of rotation may be fixed for every recording analyzed by the system.

In addition, in some embodiments, directions of the individual VCG projections may be preserved, as shown in the arrows depicted as part of each of the projections 248R-B (also visible in the two-dimensional image 250). These directions may be leveraged as additional features that are provided as inputs to a CNN to make inferences about medical conditions of subjects.

By implementing the conversions depicted in Figs. 2-4, it is possible to convert a whole dataset of ECG signals to a dataset of VCG images. Using the new dataset, it is then possible to fine-tune a pre-trained neural network such as a CNN to perform classification of a new image, in a process known as "transfer learning." Transfer learning is often faster and simpler than training a machine learning model from scratch, *e.g.,* with randomly initialized weights. Transfer learning also may require less training data to train the model to an acceptable degree, especially compared to the amount of training data required to train the model from scratch.

For example, some machine learning models may already be trained, e.g., with millions of images, to classify images into thousands of object categories (such as keyboard, coffee mug, pencil, various animals, *etc.*)*.* Such a network may have learned rich feature representations for a wide range of images. Accordingly, such a network can be commandeered and fine-tuned to, for instance, classify an ECG signal as "normal" or "abnormal."

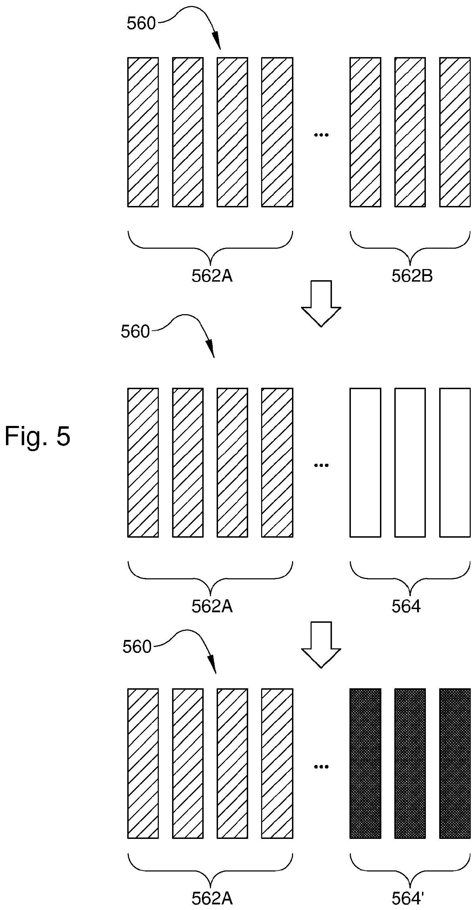

An example of this is depicted in Fig. 5. In Fig. 5 at top, a pre-trained network 560 is divided into two portions, an upstream portion 562A and a downstream portion 562B. Upstream portion 562A may include any number of hidden layers or other machine learning model components that are pre-trained to identify and/or classify relatively abstract visual features (e.g., curves, lines, patterns, *etc*.)*.* Downstream portion 562B may include hidden layers or other components that identify and/or classify features that are less abstract and/or more readily understood by humans, such as cat, dog, chair, keyboard, a handwritten character (in a handwriting recognition scenario), *etc.*

As shown in the middle of Fig. 5, downstream layers 562B may be replaced with new layer(s) 564. New layer(s) 564 are initially unshaded to indicate that they are intended to be trained to classify two-dimensional images (representative of ECG data) created using techniques described herein. In various embodiments, at this point a training dataset created from ECG signals for a population of subjects may be created and/or retrieved. In particular, techniques described herein may be employed to convert ECG signals known to be, for instance, abnormal and normal, into two-dimensional images, as described previously. These two-dimensional images may be labelled as normal or abnormal, e.g., based on medical personnel's diagnoses of the original ECG signals. These two-dimensional images may then be used to train the new network 560 (562A + 564) using the training dataset to classify an image as, for instance, normal or abnormal. As shown at bottom of Fig. 5, new layer(s) 564' are shaded to indicate that they have been trained using the ECG training dataset.

The following describes one non-limiting example of how transfer learning may be applied to leverage a preexisting model to classify ECG data (converted into two-dimensional imagery as described herein) as, for instance, normal or abnormal, or to infer other health conditions. These other health conditions may include, for instance, atrial fibrillation, heart murmur, hypertrophy, *etc.*

First, a pre-trained neural network 560 may be obtained. As shown in Fig. 5, this pre-trained network 560 may include a final three layers 562B that are trained to identify a thousand different classes of objects. These three layers may be fine-tuned and/or replaced for a new classification problem, e.g., classifying a twelve-lead, ten-second ECG recording as having either "normal" or "abnormal" morphology. All layers except the last three may be extracted from the pre-trained network. These extracted layers may be combined with three new last layers as shown at 564 in Fig. 5. In some cases these new layers 564 may include a fully connected layer, a softmax layer, and a classification output layer with two nodes corresponding to normal and abnormal.

In some embodiments, the training options and/or parameters may be set such that the new layers 564 may learn much faster than the transferred layers 562A. This may be achieved, for instance, by setting the initial learning rate for the transferred layers 562A to a smaller value compared to the newly added last three layers 564. When performing transfer learning, there may not be a need to train for as many epochs (an epoch is a full training cycle on the entire training dataset). This is because most of the network (extracted layers 562A) is already trained using a much larger training dataset.

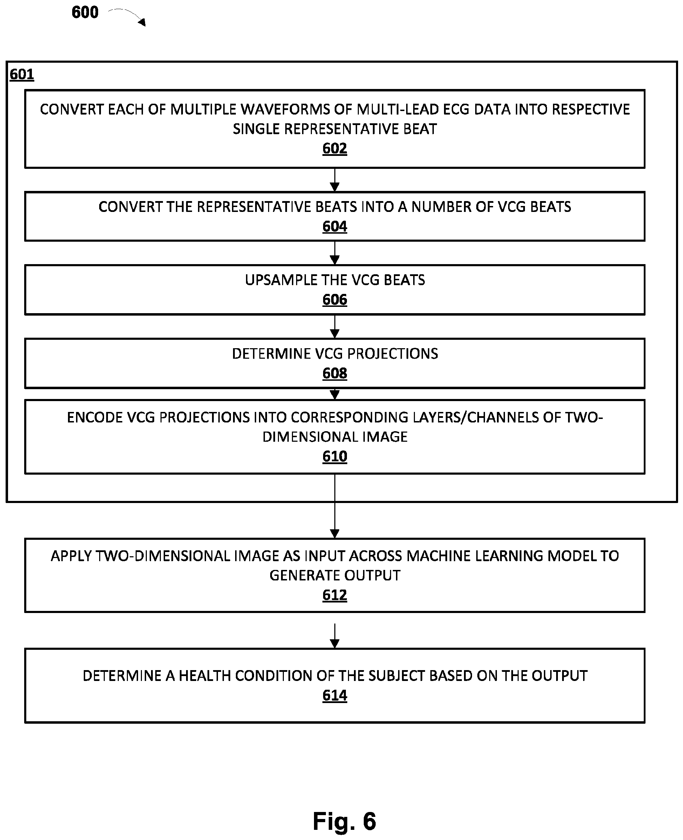

Fig. 6 illustrates a flowchart of an example method 600 for practicing selected aspects of the present disclosure. The operations of Fig. 6 can be performed by one or more processors, such as one or more processors of the various computing devices/systems described herein. For convenience, operations of method 600 will be described as being performed by a system configured with selected aspects of the present disclosure. Other implementations may include additional operations than those illustrated in Fig. 6, may perform step(s) of Fig. 6 in a different order and/or in parallel, and/or may omit one or more of the operations of Fig. 6.

At 601, which includes blocks 602-610, the system may generate a two-dimensional image based on ECG data measured from a subject. In some embodiments, the operations of block 601 may be performed by inference system 124 or training system 120 depending on the circumstances. In other embodiments, they may be performed by a separate component, not depicted in Fig. 1, such as a conversion system.

At block 602, the system may convert each of some number of waveforms of multi-lead ECG data into a respective single representative beat, as shown in Fig. 2. As noted previously, this conversion may be performed in various ways, such as averaging the beats over the ten-second time interval for each lead. At block 604, the system may convert the (*e.g.,* twelve) representative beats converted at block 602 into some number (*e.g.,* three) of VCG beats. In various embodiments, each VCG beat may correspond to a heart vector in one dimension of 3D space, as shown in Fig. 3.

In some embodiments, at block 606, the system may upsample the (*e.g.,* three) VCG beats converted at block 604, *e.g.,* to improve spatial resolution. In other embodiments, this upsampling may be omitted. At block 608, the system may determine some number (*e.g.,* three) of VCG projections based on the VCG beats. Each VCG projection may represent a respective one of the VCG beats on a spatial plane corresponding to a respective dimension of the 3D space. This was demonstrated in Fig. 4.

At block 610, the system may encode the three VCG projections into three corresponding layers or channels of a two-dimensional image. For example, one VCG projection in one spatial plane may be encoded in a red channel of an RGB digital image, another may be encoded in a green channel of the RGB image, and another may be encoded in the blue channel of the RGB image.

After blocks 602-610, the system is ready to use the generated multi-layered two-dimensional image to either make an inference (if the model is already trained), or to train the model. For example, at block 612, the system, *e.g.,* by way of inference system 124 or training system 120, may apply the multi-layered two-dimensional image as input across the machine learning model to generate output. At block 614 of Fig. 6, the system may determine a health condition of the subject based on the output. For example, the health condition may be a "normal" or "abnormal" heartbeat, *e.g.,* if the system is performing screening on a presumed healthy population.

Alternatively, the machine learning may provide more than binary output. For example, in some embodiments, the machine learning model may generate, as output, a plurality of probabilities, each associated with a different health condition. In some such embodiments, the *n* (*n*≥1) health conditions have the highest probabilities may be presented to medical personnel, *e.g.,* as part of graphical user interface, as natural language output through a speaker, as part of a report on the underlying subject, *etc.*

In other embodiments where the machine learning model is being trained, the output generated at block 612 may be compared to a label associated with the input data. For example, the input data may be ECG data that was previously labeled by medical personnel as being, for instance, "normal," abnormal," or as exhibiting one or more health conditions. The difference, or error, between the output generated using the machine learning model and the label may then be used, *e.g.,* by training system 120, to train the machine learning model.

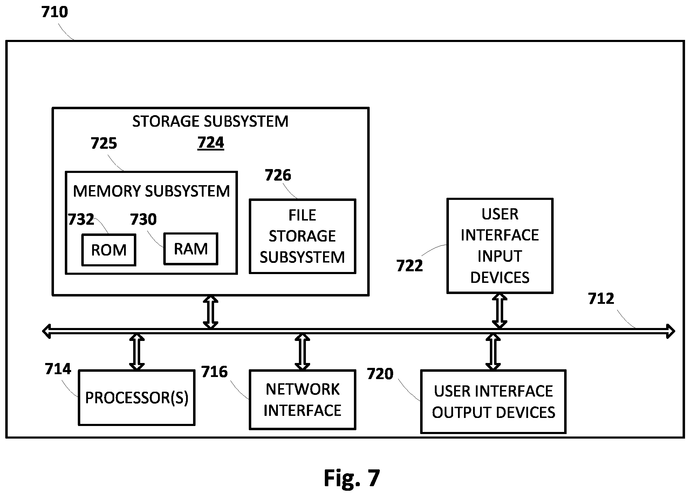

Fig. 7 is a block diagram of an example computing device 710 that may optionally be utilized to perform one or more aspects of techniques described herein. Computing device 710 typically includes at least one processor 714 which communicates with a number of peripheral devices via bus subsystem 712. These peripheral devices may include a storage subsystem 724, including, for example, a memory subsystem 725 and a file storage subsystem 726, user interface output devices 720, user interface input devices 722, and a network interface subsystem 716. The input and output devices allow user interaction with computing device 710. Network interface subsystem 716 provides an interface to outside networks and is coupled to corresponding interface devices in other computing devices.

User interface input devices 722 may include a keyboard, pointing devices such as a mouse, trackball, touchpad, or graphics tablet, a scanner, a touchscreen incorporated into the display, audio input devices such as voice recognition systems, microphones, and/or other types of input devices. In general, use of the term "input device" is intended to include all possible types of devices and ways to input information into computing device 710 or onto a communication network.

User interface output devices 720 may include a display subsystem, a printer, a fax machine, or non-visual displays such as audio output devices. The display subsystem may include a cathode ray tube (CRT), a flat-panel device such as a liquid crystal display (LCD), a projection device, or some other mechanism for creating a visible image. The display subsystem may also provide non-visual display such as via audio output devices. In general, use of the term "output device" is intended to include all possible types of devices and ways to output information from computing device 710 to the user or to another machine or computing device.

Storage subsystem 724 stores programming and data constructs that provide the functionality of some or all of the modules described herein. For example, the storage subsystem 724 may include the logic to perform selected aspects of the method of Fig. 6, as well as to implement various components depicted in Fig. 1.

These software modules are generally executed by processor 714 alone or in combination with other processors. Memory 725 used in the storage subsystem 724 can include a number of memories including a main random access memory (RAM) 730 for storage of instructions and data during program execution and a read only memory (ROM) 732 in which fixed instructions are stored. A file storage subsystem 726 can provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem 726 in the storage subsystem 724, or in other machines accessible by the processor(s) 714.

Bus subsystem 712 provides a mechanism for letting the various components and subsystems of computing device 710 communicate with each other as intended. Although bus subsystem 712 is shown schematically as a single bus, alternative implementations of the bus subsystem may use multiple busses.

Computing device 710 can be of varying types including a workstation, server, computing cluster, blade server, server farm, or any other data processing system or computing device. Due to the ever-changing nature of computers and networks, the description of computing device 710 depicted in Fig. 7 is intended only as a specific example for purposes of illustrating some implementations. Many other configurations of computing device 710 are possible having more or fewer components than the computing device depicted in Fig. 7.

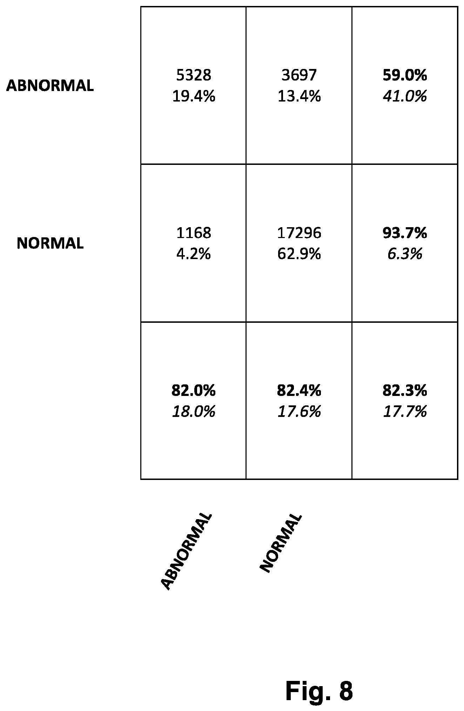

Fig. 8 depicts a confusion matrix for a "normal" versus "abnormal" classifier trained using techniques described herein. In this matrix, the rows correspond to the predicted class and the columns correspond to the true class (*e.g.,* ground truth labels). The diagonal cells correspond to observations that are correctly classified. The off-diagonal cells correspond to incorrectly classified observations. Both the number of observations and the percentage of the total number of observations are shown in each cell.

The column on the far right of the matrix shows the percentages of all the examples predicted to belong to each class that are correctly (bolded) and incorrectly (italicized) classified. These metrics are often called the precision (or positive predictive value) and false discovery rate, respectively. The row at the bottom of the plot shows the percentages of all the examples belonging to each class that are correctly and incorrectly classified. These metrics are often called the recall (or true positive rate or sensitivity) and false negative rate, respectively. The cell in the bottom right of the plot shows the overall accuracy.

The confusion matrix of Fig. 8 reveals that technique described herein, when applied to real world data, achieved 82.4% sensitivity and 93.7% positive predictive value for detecting normal ECGs. The abnormal cases in this dataset included a wide range of abnormality in ECG morphology: abnormal T wave, bundle branch block, short or prolonged PR interval, left or right ventricular hypertrophy, axis deviation, ST depression or elevation, low voltage, abnormal or missing P wave, infarct, left atrial enlargement, atrioventricular block, conduction delay, short or prolonged QT interval, poor R wave progression, ventricular preexcitation, and other abnormal morphology. This accuracy was achieved without using signal processing or any implementation of clinical rules.

While several inventive embodiments have been described and illustrated herein, those of ordinary skill in the art will readily envision a variety of other means and/or structures for performing the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims, inventive embodiments may be practiced otherwise than as specifically described.

All definitions, as defined and used herein, should be understood to control over dictionary definitions, definitions in documents incorporated by reference, and/or ordinary meanings of the defined terms. The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); *etc.*

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); *etc.*

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively. It should be understood that certain expressions and reference signs used in the claims pursuant to Rule 6.2(b) of the Patent Cooperation Treaty ("PCT") do not limit the scope.

## Claims

1. A method implemented using one or more processors, comprising:
generating (601) a two-dimensional image based on vectorcardiography ("VCG") data, wherein the VCG data is based on electrocardiogram ("ECG") data measured from a subject, wherein the ECG data comprises multiple waveforms corresponding to multiple ECG leads, and generating the image comprises:
converting (602) each of the multiple waveforms into a respective single representative beat, wherein the single representative beat for each waveform is an average of all beats comprised by the waveform,
converting (604) the multiple representative beats into three VCG beats, wherein each VCG beat corresponds to heart vector values in one dimension of three-dimensional ("3D") space, the three VCG beats therefore together defining a 3D VCG signal, consisting of a continuous series of 3D vectors forming a curved line in three dimensional space,
determining (608) three VCG projections, each VCG projection representing a projection of the said 3D VCG signal onto a respective spatial plane corresponding to one of the dimensions of the 3D space, and
encoding (610) the three VCG projections into three corresponding layers of the two-dimensional image;
applying (612) the two-dimensional image as input across a machine learning model to generate an output, wherein the machine learning model is a convolutional neural network (CNN) configured for use in processing two-dimensional images;
and
determining (614) a health condition of the subject based on the output.

2. The method of claim 1, further comprising upsampling (606) the three VCG beats.

3. The method of claim 1, wherein the three corresponding layers comprise red, green, and blue.

4. A device comprising a processor and memory, wherein the memory stores instructions that, in response to execution of the instructions by the processor, cause the device to:
generate (601) a two-dimensional image based on vectorcardiography ("VCG") data, wherein the VCG data is based on electrocardiogram ("ECG") data measured from a subject;
wherein the ECG data comprises multiple waveforms corresponding to multiple ECG leads, and generating the image comprises:
converting (602) each of the multiple waveforms into a respective single representative beat, wherein the single representative beat for each waveform is an average of all beats comprised by the waveform,
converting (604) the multiple representative beats into three VCG beats, wherein each VCG beat corresponds to heart vector values in one dimension of three-dimensional ("3D") space, the three VCG beats therefore together defining a 3D VCG signal, consisting of a continuous series of 3D vectors forming a curved line in three dimensional space,
determining (608) three VCG projections, each VCG projection representing a projection of the said 3D VCG signal onto a respective spatial plane corresponding to one of the dimensions of the 3D space, and
encoding (610) the three VCG projections into three corresponding layers of the two-dimensional image;
apply (612) the two-dimensional image as input across a machine learning model to generate an output, wherein the machine learning model is a convolutional neural network configured for use in processing two-dimensional images;
and
determine (614) a health condition of the subject based on the output.

5. The device of claim 4, further comprising instructions to upsample (606) the plurality of VCG beats.

6. At least one non-transitory computer-readable medium comprising instructions that, in response to execution of the instructions by one or more processors, cause the one or more processors to perform the method of claim 1.

## Patentansprüche

1. Verfahren, das unter Verwendung eines oder mehrerer Prozessoren umgesetzt wird und Folgendes umfasst:
Erzeugen (601) eines zweidimensionalen Bildes basierend auf Vektorkardiographie-("VCG")-Daten, wobei die VCG-Daten auf Elektrokardiogramm-("EKG")-Daten basieren, die von einem Subjekt gemessen wurden, wobei die EKG-Daten mehrere Wellenformen umfassen, die mehreren EKG-Ableitungen entsprechen, und Erzeugen des Bildes Folgendes umfasst:
Konvertieren (602) jeder der mehreren Wellenformen in einen jeweiligen einzelnen repräsentativen Schlag, wobei der einzelne repräsentative Schlag für jede Wellenform ein Durchschnitt aller in der Wellenform enthaltenen Schläge ist,
Konvertieren (604) der mehreren repräsentativen Schläge in drei VCG-Schläge, wobei jeder VCG-Schlag Herzvektorwerten in einer Dimension von dreidimensionalem ("3D") Raums entspricht, wobei die drei VCG-Schläge dafür zusammen ein 3D-VCG-Signal definieren, das aus einer kontinuierlichen Reihe von 3D-Vektoren besteht, die eine gekrümmte Linie im dreidimensionalen Raum bilden,
Bestimmen (608) von drei VCG-Projektionen, wobei jede VCG-Projektion eine Projektion des 3D-VCG-Signals auf eine jeweilige räumliche Ebene darstellt, die einer der Dimensionen des 3D-Raums entspricht, und
Kodieren (610) der drei VCG-Projektionen in drei entsprechende Ebenen des zweidimensionalen Bildes;
Anwenden (612) des zweidimensionalen Bildes als Eingabe über ein Modell des maschinellen Lernens zur Erzeugung einer Ausgabe, wobei es sich bei dem Modell des maschinellen Lernens um ein neuronales Faltungsnetzwerk (CNN) handelt, das für Verwendung bei Verarbeitung zweidimensionaler Bilder konfiguriert ist;
und
Bestimmen (614) eines Gesundheitszustands des Subjekts basierend auf der Ausgabe.

2. Verfahren nach Anspruch 1, weiter umfassend Aufwärtsabtastung (606) der drei VCG-Schläge.

3. Verfahren nach Anspruch 1, wobei die drei entsprechenden Schichten rot, grün und blau umfassen.

4. Vorrichtung, umfassend einen Prozessor und einen Speicher, wobei der Speicher Anweisungen speichert, die, als Reaktion auf Ausführung der Anweisungen durch den Prozessor die Vorrichtung veranlassen zum:
Erzeugen (601) eines zweidimensionalen Bilds basierend auf Vektorkardiographie-("VCG")-Daten , wobei die VCG-Daten auf Elektrokardiogramm-("EKG")-Daten basieren, die von einem Subjekts gemessen wurden;
wobei die EKG-Daten mehrere Wellenformen umfassen, die mehreren EKG-Ableitungen entsprechen, und Erzeugen des Bildes Folgendes umfasst:
Konvertieren (602) jeder der mehreren Wellenformen in einen jeweiligen einzelnen repräsentativen Schlag, wobei der einzelne repräsentative Schlag für jede Wellenform ein Durchschnitt aller in der Wellenform enthaltenen Schläge ist,
Konvertieren (604) der mehreren repräsentativen Schläge in drei VCG-Schläge, wobei jeder VCG-Schlag Herzvektorwerten in einer Dimension von dreidimensionalem ("3D") Raums entspricht, wobei die drei VCG-Schläge dafür zusammen ein 3D-VCG-Signal definieren, das aus einer kontinuierlichen Reihe von 3D-Vektoren besteht, die eine gekrümmte Linie im dreidimensionalen Raum bilden,
Bestimmen (608) von drei VCG-Projektionen, wobei jede VCG-Projektion eine Projektion des 3D-VCG-Signals auf eine jeweilige räumliche Ebene darstellt, die einer der Dimensionen des 3D-Raums entspricht, und
Kodieren (610) der drei VCG-Projektionen in drei entsprechende Ebenen des zweidimensionalen Bildes;
Anwenden (612) des zweidimensionalen Bildes als Eingabe über ein Modell des maschinellen Lernens zur Erzeugung einer Ausgabe, wobei es sich bei dem Modell des maschinellen Lernens um ein neuronales Faltungsnetzwerk handelt, das für Verwendung bei Verarbeitung zweidimensionaler Bilder konfiguriert ist;
und
Bestimmen (614) eines Gesundheitszustands des Subjekts basierend auf der Ausgabe.

5. Vorrichtung nach Anspruch 4, weiter umfassend Anweisungen für Aufwärtsabtastung (606) der Vielzahl von VCG-Schlägen.

6. Mindestens ein nichtflüchtiges, computerlesbares Medium, das Anweisungen umfasst, die als Reaktion auf Ausführung der Anweisungen durch einen oder mehrere Prozessoren bewirken, dass der eine oder die mehreren Prozessoren das Verfahren nach Anspruch 1 durchführen.

## Revendications

1. Procédé mis en œuvre à l'aide d'un ou plusieurs processeurs, comprenant :
la génération (601) d'une image bidimensionnelle sur la base de données de cardiographie vectorielle (« VCG »), dans lequel les données VCG sont basées sur des données d'électrocardiogramme (« ECG ») mesurées chez un sujet, dans lequel les données ECG comprennent de multiples formes d'onde correspondant à de multiples dérivations ECG, et la génération de l'image comprend :
la conversion (602) de chacune des multiples formes d'onde en un unique battement représentatif respectif, dans lequel l'unique battement représentatif pour chaque forme d'onde est une moyenne de tous les battements compris par la forme d'onde,
la conversion (604) des multiples battements représentatifs en trois battements VCG, dans lequel chaque battement VCG correspond à des valeurs vectorielles cardiaques dans une dimension d'espace tridimensionnel (« 3D »), les trois battements VCG définissant donc conjointement un signal VCG 3D, constitué d'une série continue de vecteurs 3D formant une ligne courbe dans l'espace tridimensionnel,
la détermination (608) de trois projections VCG, chaque projection VCG représentant une projection dudit signal VCG 3D sur un plan spatial respectif correspondant à l'une des dimensions de l'espace 3D, et
le codage (610) des trois projections VCG dans trois couches correspondantes de l'image bidimensionnelle ;
l'application (612) de l'image bidimensionnelle comme entrée à travers un modèle d'apprentissage automatique pour générer une sortie, dans lequel le modèle d'apprentissage automatique est un réseau neuronal convolutif (CNN) configuré pour être utilisé dans le traitement d'images bidimensionnelles ;
et
la détermination (614) d'un état de santé du sujet sur la base de la sortie.

2. Procédé selon la revendication 1, comprenant en outre un suréchantillonnage (606) des trois battements VCG.

3. Procédé selon la revendication 1, dans lequel les trois couches correspondantes comprennent du rouge, du vert et du bleu.

4. Dispositif comprenant un processeur et une mémoire, dans lequel la mémoire stocke des instructions qui, en réponse à l'exécution des instructions par le processeur, amènent le dispositif à :
générer (601) une image bidimensionnelle sur la base de données de cardiographie vectorielle (« VCG »), dans lequel les données VCG sont basées sur des données d'électrocardiogramme (« ECG ») mesurées chez un sujet ;
dans lequel les données ECG comprennent de multiples formes d'onde correspondant à de multiples dérivations ECG, et la génération de l'image comprend :
la conversion (602) de chacune des multiples formes d'onde en un unique battement représentatif respectif, dans lequel l'unique battement représentatif pour chaque forme d'onde est une moyenne de tous les battements compris par la forme d'onde,
la conversion (604) des multiples battements représentatifs en trois battements VCG, dans lequel chaque battement VCG correspond à des valeurs vectorielles cardiaques dans une dimension d'espace tridimensionnel (« 3D »), les trois battements VCG définissant donc conjointement un signal VCG 3D, constitué d'une série continue de vecteurs 3D formant une ligne courbe dans l'espace tridimensionnel,
la détermination (608) de trois projections VCG, chaque projection VCG représentant une projection dudit signal VCG 3D sur un plan spatial respectif correspondant à l'une des dimensions de l'espace 3D, et
le codage (610) des trois projections VCG dans trois couches correspondantes de l'image bidimensionnelle ;
appliquer (612) l'image bidimensionnelle comme entrée à travers un modèle d'apprentissage automatique pour générer une sortie, dans lequel le modèle d'apprentissage automatique est un réseau neuronal convolutif configuré pour être utilisé dans le traitement d'images bidimensionnelles ;
et
déterminer (614) un état de santé du sujet sur la base de la sortie.

5. Dispositif selon la revendication 4, comprenant en outre des instructions pour suréchantillonner (606) la pluralité de battements VCG.

6. Au moins un support non transitoire lisible par ordinateur comprenant des instructions qui, en réponse à l'exécution des instructions par un ou plusieurs processeurs, amènent les un ou plusieurs processeurs à réaliser le procédé de la revendication 1.
